# EUROPEAN PATENT APPLICATION

(11) **EP 1 669 344 A1**
(43) Date of publication of application: **14.06.2006**
(21) Application number: 04748574.3
(22) Date of filing: 06.08.2004
(51) Int. Cl.: C07C 66/02

(54) **METHOD FOR THE PURIFICATION OF CRUDE DIACEREIN BY MEANS OF TOLUENE**

(30) Priority: 23.09.2003 MX PA03008622
(71) Applicant: Interquim, S.A. De C.V., C.P. 54730, Estado de Mexico (MX)
(72) Inventor: CASAZZA FANCHINI, Umberto, Bruno, C.P. 54054, Tlanepantla, Estado de Méxic (MX)
(74) Representative: Thom, Russell
(86) International application number: PCT/MX2004/000058
(87) International publication number: WO 2005/028412

(57) **Abstract**

A method for purifying crude diacerein by means of the use of toluene which consists of: dissolving crude diacerein in about 13 times the volume of dry diacerein of a 1:1 acetone/water mixture; adjusting the pH at 6.6-7.2, preferably 7.0-7.2, with a solution of a tertiary amine in acetone; a trialkylamine with C1-4 alkyl groups may be used as a tertiary amine, preferably selected from the group: trimethylamine, triethylamine, tripropylamine, methyldiethylamine, diethylpropylamine, among which triethylamine is most preferred; stirring until the diacerein is completely dissolved; adding an organic water immiscible-solvent and stir; performing between 5 and about 15 repeated extractions with the organic solvent and each time separating the organic phase; diacerein is crystallized in the acetone/water phase when changing the pH from neutral to acid with a strong mineral acid selected from hydrochloric, sulphuric and phosphoric acid; the crystallized product is centrifuged or filtered, washed with water and dried. Following this method diacerein is obtained under a 90-93% yield, an average purity of 99.17%, a content of aloe-emodin of 7-10 ppm and a content of chromium of 20-25 ppm.

## Description

### FIELD OF THE INVENTION

This invention is associated to purification methods for substances that are insoluble or slightly soluble in water, and which will be used as an active ingredient in a medicine product, particularly, it is associated to the purification of diacerein.

### BACKGROUND OF THE INVENTION

Anthraquinones and their derivatives are useful to treat the symptoms of arthritis; particularly, 1,8-dihydroxy and 1,8-diacetoxy anthraquinones, especially 1,8-diacetoxy-3-carboxy-anthraquinone, known as diacerein.

These compounds have a drawback: they are not soluble in water at all or they are slightly soluble in water. This makes their purification difficult and, therefore, various purification methods have been proposed in order to obtain a product that is pure and soluble in water.

The synthesis of diacerein is carried out by the oxidation of aloe-emodin with hexavalent chromium according to "Pharmazeutische Wirkstoffe, Synthesen, Patente Anwedungen", George Thieme Verlag, Stuttgart-New York, 1982-1987; or by the oxidation of triacyl aloe-emodin obtained by the acetylation of aloe-emodin.

European Patent 928 781 B1, granted to Laboratoire Medidom S.A., on March 27^{th}, 2002, entitled "Process for the preparation of rhein and its diacyl derivatives", protects a process that uses toluene to purify, in this case, triacetyl aloe-emodin.

Triacetyl aloe-emodin is chemically different from diacerein because it shows a hydroxyl group instead of the carboxyl that is present in diacerein. In Patent EP 928 781 B1, aloe-emodin is obtained by synthesis with a ferric acid, for example, FeCl₃; which is different from a synthesis with chromium VI. In addition, triacetyl aloe-emodin is an intermediate in the synthesis of diacerein. The method of this invention is used to purify diacerein.

In the aforementioned European patent, triacetyl aloe-emodin is purified by crystallization using toluene, alone or mixed with methanol; which is heated until completely dissolved and allowed to cool down until it precipitates; then, it is crystallized once more with a mixture of toluene/methanol, it is heated and allowed to cool down until it crystallizes.

The first disadvantage of this method is the direct dissolution of the active compound in toluene; this involves the use of toluene in solid triacetyl aloe-emodin, which does not enable the effective extraction of the compound. On the other hand, only two extractions are carried out.

The technical problem is that, given the synthesis method via the chromium VI pathway, diacerein is contaminated mainly with chromium; some amount of that chromium, as well as some of the metal that remains trapped in the diacerein crystals, is eliminated with the current methods to purify diacerein. Chromium pollutes the environment and it is toxic to organisms, including human beings. Therefore, a purification method that provides a high degree of purity and highly water-soluble diacerein is necessary.

The international application number PGT/EP01/06019, bearing international publication number WO 01/96276 A1, entitled "A process for the purification of diacerein", applied for by SYNTECO SPA and published on December 20^{th} 2001, claims a process to purify diacerein in which crude diacerein is dissolved in hot acetic anhydride/acetic acid or acetic anhydride alone, ethylene diamine tetra acetic acid (EDTA) is added to remove the released chromium from the diacerein synthesis; filtration is performed while the mixture is hot, after that, it is allowed to cool down and diacerein is crystallized. According to this patent application, a product with a purity of 99.8%, containing less than 70 ppm aloe-emodin and less than 15 ppm chromium, as analyzed by atomic absorption' is obtained.

The method described in the patent application WO 01/96276 has the disadvantage that, if the diacerein dissolved while hot and the EDTA are filtered while hot, the EDTA also drags diacerein, reducing the total yield; on the other hand, if the filtrate is performed only once, an amount of aloe-emodin up to 70 ppm remains at the end.

The international application number PCT/EO00/03691, international publication number WO 00/68179 A1, entitled "A process for the purification of diacerein", applied for by SYNTECO SPA and published on November 16^{th}, 2000, claims a process to purify diacerein. The first part of this process is very similar to the aforementioned application, crude diacerein is dissolved in a mixture of hot acetic anhydride/acetic acid or hot acetic anhydride alone, EDTA is added to remove the chromium, it is filtered while hot and then, it is allowed to cool down, filtered or centrifuged. In the second part of the process, the filtrate is washed with acetic acid and, after that, with water; triethylamine dissolved in acetone is added and the mixture is precipitated with phosphoric acid in a 4-8% aqueous solution, it is washed with water and dried. According to this patent application, diacerein in an 80-90% yield, with a minimum purity of 99.7%, less than 70 ppm aloe-emodin and with less than 15 ppm chromium is obtained.

Unlike international applications WO 01/96276 and WO 00/68179, no EDTA is used in the invention of this application. Diacerein with a weight/weight yield of 90-93%, with an average purity of 99.17%, containing 7-10 ppm of aloe-emodin and 20-25 ppm of chromium is obtained, which is an advantage over the state of the art.

European patent EP 754 173 B1, granted based on international application number PCT/IB96/00093, international publication number WO 96/24572, entitled "Method For Purifying Diacetyl Rhein", applied for by STEBA BEHEER B.V. and published on April 14^{th}, 1999, claims a process to purify diacerein which consists of suspending crude diacerein in an organic solvent and water, the solvent may be acetone, methylketone, ethanol or diethylacetamide; a solution is obtained by adding a tertiary amine, after that, diacerein is precipitated as the salt of an alkaline metal or alkaline earth metal. Finally, it is dissolved in water to obtain the diacerein in an acid medium. However, dissolving the diacerein salt in water before the acidification generates partial deacetylation with the subsequent formation of impurities that cause a loss of about 0.5-5% in the final amount of the product, which is statistically equivalent to a 2% loss.

On the other hand, it is known that the protection of hydroxy groups by acetylation is reversible; hydrolysis takes place even in slightly acid or basic media. Diacerein remains stable when it goes from neutrality to acidity, but it is quickly deacetylated even in a slightly basic medium and the basicity produced by the diacerein sodium salt is enough to generate hydrolysis. This way, any later acetylation is complex, incomplete and causes a loss of product.

The main difference between the European patent EP 754 173 B1 method and the method of this invention is that; in the first method, diacerein is precipitated as the sodium salt with the aforementioned disadvantages; while, in the method of this invention, diacerein is purified by repeated dissolution and precipitation.

The international application PCT/EP98/03221, under international publication number WO 98/56750, published on December 17^{th}, 1998, claims a method to obtain and purify diacerein including the following steps:
a) acetylation of aloin to produce acetyl barbaloin
b) oxidation of acetyl barbaloin to produce crude diacerein
c) salification of crude diacerein with an organic amine in water or acetone/water solution.
d) precipitation of the alkaline salt by adding an alkaline salt of an organic acid and treating with a diluted acid to produce acid diacerein

The disadvantage of this technique is that, the product is precipitated after the salification step, which does not help to separate the chromium formed during the synthesis, additionally, the aforementioned is applicable only to acetylation and basic and acid hydrolysis. The invention hereof helps to eliminate the chromium and the aloe-emodin by performing dissolutions and precipitations of diacerein.

### BACKGROUND OF THE INVENTION

1- To provide a method for purifying diacerein with a high degree of purity.
2- To provide a method to purify and to obtain diacerein with an average purity of 99.17%
3- To provide a method to reduce the concentration of aloe-emodin to a maximum of 10 ppm.
4- To provide a method to reduce the concentration of chromium to a maximum of 25 ppm.
5- To provide a method in which diacerein with a 90-93% yield by weight can be obtained after purification.

### DETAILED DESCRIPTION OF THE INVENTION

The method for purification of crude diacerein by means of toluene consists of the following steps:

### Steps of the purification method:

a) Crude diacerein is dissolved in acetone/water
b) The pH is adjusted with a tertiary amine in acetone
c) The solution is stirred for about six hours
d) An organic water immiscible-solvent is added and the solution is stirred
e) The organic solvent phase is separated from the acetone/water phase
f) The extraction is repeated about 5 to 15 times with the water-immiscible organic solvent and, every time, the organic phase is separated from the acetone/water phase
g) The organic phase is continuously separated and diacerein is crystallized from the acetone/water phase when the pH changes from neutral to acid with a strong acid
h) The crystallized product is centrifuged or filtered, washed with water and dried

Crude diacerein obtained by acetylation and chromium oxidation is dissolved, while wet, in a mixture of 1/1 acetone/water mixture in a proportion of about 13 times the volume of the dry diacerein; the pH is adjusted from about 6.6-7.2 to preferably 7.0-7.2 with a tertiary amine in acetone solution and, in about six hours, complete dissolution is achieved; a trialkylamine with C1-4 alkyl groups may be used as a tertiary amine, preferably from the group: trimethylamine, triethylamine, tripropylamine, methyldiethylamine, diethylpropylamine, among which triethylamine is most preferred.

Once the diacerein is completely dissolved, it is purified by organic extraction with a water immiscible-solvent selected from the group consisting of: benzene, toluene, xylene, isobutyl acetate and ethyl acetate, among which toluene is most preferred.

By using the organic water immiscible-solvent at the time diacerein is completely dissolved in the mixture of tertiary amine/acetone, the diacerein extraction is favored and the disadvantage of dissolving the active compound directly in the organic solvent is eliminated; this means that if, for example, toluene is selected and used with solid diacerein, it does not help to achieve an efficient extraction because a considerable amount of the contaminants does not dissolve in toluene and it remains trapped in the diacerein crystals.

Surprisingly, it was found that a higher amount of chromium, aloe-emodin and other impurities trapped in the diacerein crystals is released with a larger number of extractions and; therefore, diacerein with a higher degree of purity is obtained.

To perform the extraction of diacerein with toluene: a volume of toluene equivalent to 1.6 volumes of dry diacerein is added to the dissolution; the dissolution is stirred and the toluene phase is separated from the acetone/water solution; about 5 to 15, preferably a minimum of 10, repeated extractions are performed with the same amount of toluene and with the same proportion of toluene to dry diacerein. Surprisingly, it has been found that, when more extractions with toluene are performed, a product with a higher degree of purity and, therefore, with a lower concentration of contaminants is obtained.

After performing at least 10, preferably 15, extractions with toluene, diacerein is crystallized at a pH of about 2.5 in water and at a pH of about 3.0 in a strong acid, such as sulfuric, hydrochloric or phosphoric acid; 80% phosphoric acid is preferred. It is centrifuged or filtered, then, washed with water and dried.

In this method, the extraction is liquid/liquid, which makes it more efficient than a liquid/solid extraction: For this reason, it is important to dilute the diacerein before adding the organic solvent instead of dissolving the diacerein directly in the organic solvent.

Diacerein alkaline salts are insoluble in the acetone/water mixture. The alkaline salt of triethylamine is insoluble or partially soluble in water but it dissolves easily in the acetone/water mixture. This is the reason why the triethylamine salt is dissolved in acetone/water first and, then, the diacerein is dissolved in said solution; without this, a good yield of diacerein or a high degree of purity cannot be guaranteed.

One of the most important impurities found in crude diacerein after chromium oxidation is aloe-emodin; which is a molecule similar to diacerein, the difference between them is that one has an alcohol group and the other has a carboxyl group, which, in fact, is an incomplete oxidation of the alcohol group.

Another important impurity is chromium, which may be free in the form of a chromium salt (III), or in combination with diacerein. Diacerein forms complexes with some metals such as Mg, Ca, Fe, Cr, etc.

Being the salt of a tertiary amine dissolved in the adequate medium, diacerein allows the extraction of impurities from its crystals. The impurities are present in the dissolution before the crystals are formed, so the impurities are not trapped in the crystals, the same for non-salifiable impurities such as aloe-emodin. Diacerein combined with chromium and chromium (III) passes easily into the extraction solvent, which also extracts most of the acetone in the dissolution mixture.

The continuous and repeated extraction with unmixed solvent allows removal of the impurities through that solvent, and their elimination by separation of the solvent from the water phase.

Part of the diacerein passes through the separated solvent and it can be easily retrieved by changing the pH from neutral to acid, by simply adding a strong acid to the solvent.

Using this method, a 90-93% weight/weight yield containing 7-10 ppm of aloe-emodin and 20-25 ppm of chromium is generally obtained after purification.

To reduce the contents of impurities even more, this procedure may be repeated or the number of extractions may be increased.

### Example #1

100 kg of crude humid diacerein (equivalent to 62 kg of dry diacerein), are dissolved at a maximum pH between 7.0-7.2 in a mixture of 375.8 kg of water and 441.2 kg of acetone; 16.33 kg of triethylamine dissolved in 186.2 kg of acetone at a temperature of 23-25°C for 6-8 hours are required.

when diacerein has dissolved completely, 10 extractions are performed with 100 liters of toluene, using 10 liters in every extraction. After that, the water phase is adjusted to a pH of about 2.8 with 80% phosphoric acid and a 91-92.5% weight/weight yield, 8 ppm of aloe-emodin, 23 ppm of chromium, 99.24% purity and a 98.55% concentration is obtained.

### Example #2

In the same procedure of example 1, after the dissolution, 10 extractions with 100 liters of isobutyl acetate, using 10 liters in every extraction are performed; after crystallization at a pH of about 2.5, using 10% sulphuric acid, a 92-93% weight/weight yield, 9 ppm of aloe-emodin, 23 ppm of chromium, a purity of 99.24% and a concentration of 98.55% are obtained.

### Example #3

In the same procedure of example 1, after the dissolution, 10 extractions with 100 liters of xylene, using 10 liters in every extraction are performed; after crystallization at a pH of about 3.0, using 30% phosphoric acid, a 90-92% weight/weight yield, 8 ppm of aloe-emodin, 24 ppm of chromium, a purity of 98.76% and a concentration of 98.99% are obtained.

### Example #4

In the same procedure of example 1, after the dissolution, 10 extractions with 100 liters of toluene, using 10 liters in every extraction are performed; after crystallization at a pH of about 2.8, using 10% sulphuric acid, a 90-93% weight/weight yield, 7 ppm of aloe-emodin, 20 ppm of chromium, a purity of 99.31% and a concentration of 99.44% are obtained.

### Example #5

In the same procedure of example 1, after the dissolution, 10 extractions with 100 liters of xylene, using 10 liters in every extraction are performed; after crystallization at a pH of about 2.7, using 10% sulphuric acid, a 91-93% weight/weight yield, 7 ppm of aloe-emodin, 21 ppm of chromium, a purity of 99.31% and a concentration of 99.44% are obtained.

### Example #6

In the same procedure of example 1, after the dissolution, 10 extractions with 100 liters of ethyl acetate, using 10 liters in every extraction are performed; after crystallization at a pH of about 2.2, using 80% phosphoric acid, a 88-90% weight/weight yield, 10 ppm of aloe-emodin, 25 ppm of chromium, a purity of 99.16% and a concentration of 98.96% are obtained.

## Claims

1. A method for purifying crude diacerein by means of toluene, wherein the diacerein is obtained by acetylation and chrome oxidation, **characterized by** comprising the following steps:
a) crude diacerein is dissolved in acetone/water,
b) the pH is adjusted with a tertiary amine in acetone,
c) the solution is stirred for about six hours,
d) an organic water immiscible-solvent is added and the solution is stirred,
e) the organic solvent phase is separated from the acetone/water phase,
f) the extraction is repeated about 5 to 15 times with the organic water immiscible-solvent and, every time, the organic phase is separated from the acetone/water phase,
g) the diacerein is crystallized from the acetone/water phase upon changing the pH from neutral to acid with a strong acid,
h) the crystallized product is centrifuged or filtered, washed with water and dried.

2. A method for purifying crude diacerein by means of toluene, wherein the diacerein is obtained by acetylation and chrome oxidation, according to claim 1, **characterized in that** in step a), crude diacerein is dissolved, while wet, in a 1/1 acetone/water mixture in an proportion of about 13 times the volume of dry diacerein, and it is stirred until completely dissolved.

3. A method for purifying crude diacerein by means of toluene, wherein the diacerein is obtained by acetylation and chrome oxidation, according to claim 2, **characterized in that** in step b), the pH is adjusted at 6.6-7.2, preferably 7.0-7.2, with a solution of a tertiary amine in acetone.

4. A method for purifying crude diacerein by means of toluene, wherein the diacerein is obtained by acetylation and chrome oxidation, according to claim 3, **characterized in that** a trialkylamine with C1-4 alkyl groups may be used as a tertiary amine, preferably selected from the group: trimethylamine, triethylamine, tripropylamine, methyldiethylamine, diethylpropylamine, among which triethylamine is most preferred.

5. A method for purifying crude diacerein by means of toluene, wherein the diacerein is obtained by acetylation and chrome oxidation, according to claim 2, **characterized in that** in step c), stirring lasts about six hours in order for the diacerein to dissolve completely.

6. A method for purifying crude diacerein by means of toluene, wherein the diacerein is obtained by acetylation and chrome oxidation, according to claim 1, **characterized in that** in step d), once the diacerein is completely dissolved, it is purified by organic extraction with a water immiscible-solvent , which is selected from the group consisting of: benzene, toluene, xylene, isobutyl acetate and ethyl acetate, among which toluene is most preferred.

7. A method for purifying crude diacerein by means of toluene, wherein the diacerein is obtained by acetylation and chrome oxidation, according to claim 1, **characterized in that** in step e) the extraction of diacerein with the organic water immiscible-solvent is performed, a volume of toluene equivalent to 1.6 volumes of the dry diacerein is added to the dissolution; the dissolution is stirred and the organic phase is separated from the acetone/water solution'; about 5 to 15 repeated extractions (step f) are performed with the same amount of organic water immiscible-solvent and with the same proportion of the solvent to dry diacerein.

8. A method for purifying crude diacerein by means of toluene, wherein the diacerein is obtained by acetylation and chrome oxidation, according to claim 7, **characterized in that** a minimum of 10 repeated extractions is preferred.

9. A method for purifying crude diacerein by means of toluene, wherein the diacerein is obtained by acetylation and chrome oxidation, according to claim 7, **characterized in that** about 15 repeated extractions with the organic solvent are preferably performed.

10. A method for purifying crude diacerein by means of toluene, wherein the diacerein is obtained by acetylation and chrome oxidation, according to claim 1, **characterized in that** in step g), after at least 10 extractions with the organic water immiscible-solvent, the diacerein is crystallized in water at a pH between about 2.5 and about 3.0 with a strong acid, such as sulphuric, hydrochloric or phosphoric acid.

11. A method for purifying crude diacerein by means of toluene, wherein the diacerein is obtained by acetylation and chrome oxidation, according to claim 10, **characterized in that** 80% phosphoric acid is preferred.

12. A method for purifying crude diacerein by means of toluene, wherein the diacerein is obtained by acetylation and chrome oxidation, according to claim 1, **characterized in that** in step h), it is centrifuged or filtered, washed with water and dried.

13. A method for purifying crude diacerein by means of toluene, wherein the diacerein is obtained by acetylation and chrome oxidation, according to claim 1,
**characterized in that** diacerein is obtained under a 90-93% yield, with an average purity of 99.17%, a content of aloe-emodin of about 7-10 ppm and a content of chromium of about 20-25 ppm.
